# EUROPEAN PATENT APPLICATION

(11) **EP 4 378 472 A1**
(43) Date of publication of application: **05.06.2024**
(21) Application number: 22849615.4
(22) Date of filing: 29.07.2022
(51) Int. Cl.: A61K 38/17, A61K 48/00, A61P 1/00, A61P 9/10, A61P 11/00, A61P 29/00, C07K 14/47, C12N 15/12

(54) **THERAPEUTIC AGENT FOR ISCHEMIC DISEASE**

(30) Priority: 30.07.2021 JP 2021125789
(71) Applicant: Miyazaki, Toru, Tokyo 162-8666 (JP)
(72) Inventor: Miyazaki, Toru, Tokyo 162-8666 (JP)
(74) Representative: Weickmann & Weickmann PartmbB
(86) International application number: PCT/JP2022/029282
(87) International publication number: WO 2023/008555

(57) **Abstract**

The present invention provides an agent for treating an ischemic disease, including an apoptosis inhibitor of macrophage (AIM), an AIM fragment having a biological activity of AIM, or a nucleic acid encoding the AIM or AIM fragment.

## Description

### [Technical Field]

The present invention relates to a therapeutic agent for ischemic diseases and the like. More particularly, the present invention relates to a therapeutic agent for ischemic diseases, including apoptosis inhibitor of macrophage (AIM), and the like.

### [Background Art]

The inefficient removal of dead cells and their debris results in the release of intracellular inflammatory agents, so-called damage-associated molecular patterns (DAMPs). While DAMPs are normal endogenous intracellular proteins, and therefore hidden from recognition by the immune system, once released into the extracellular environment, they bind to pattern recognition receptors, including Toll-like receptors (TLRs) of immunocytes such as macrophage and receptor for advanced glycation end-products, and activate innate immunity to induce the production of inflammatory cytokines in the absence of microorganisms. This process is called sterile inflammation, which is often caused by ischemia-reperfusion injury (IRI), trauma, or chemically induced injury (Non Patent Literature 1).

Ischemic stroke, one of the most common causes of severe disability and death globally, and currently featured as an important complication of COVID-19 is typically associated with sterile inflammation (Non Patent Literatures 2 to 5). There is a widely accepted consensus that the prognosis of ischemic stroke is highly influenced by the state of sterile inflammation in the affected brain after the onset of stroke. Since currently available therapies, such as thrombolysis and thrombectomy, do not always profoundly improve the prognosis of stroke patients, increasing attention has recently been paid to post-stroke sterile inflammation as a therapeutic target (Non Patent Literatures 5 to 7). Of the different types of DAMPs, recent evidence shows that peroxiredoxin (PRDX; particularly PRDX1), high-mobility-group-box 1 (HMGB1), and S100 calcium-binding proteins (S100) are highly involved in post-stroke sterile inflammation (Non Patent Literature 8). More recently, it has been reported that as a repair process, DAMPs are internalized by microglia and macrophages infiltrating an infarct area through macrophage scavenger receptor 1, whose expression is regulated by MAF bZIP transcription factor B (MafB). However, no effective stroke treatments based on DAMP removal have been established.

Apoptosis inhibitor of macrophage (AIM; also called CD5 antigen-like: CD5L) is a circulating protein produced by tissue macrophages, which the present inventors initially identified as a supporter of macrophage survival. AIM is now recognized as a molecule to induce repair processes in many diseases (Non Patent Literatures 10 to 12). AIM consists of three cysteine-rich domains (termed the SRCR domains), and a unique positively-charged amino-acid cluster is present at the carboxyl terminus within the third SRCR domain. This cluster develops charge-based interactions with dead cells, whose surface is strongly negatively charged due to the high levels of exposed phosphatidylserine (Non Patent Literatures 10, 11). This association highly enhances the engulfment of dead cells by phagocytes, as AIM is highly internalized by phagocytes via multiple scavenger receptors (Non Patent Literature 12). The present inventors assess the possible impact of AIM on the pathophysiology of ischemic stroke, where the main pathophysiology is partial necrosis of brain neurons due to transient ischaemia and sterile inflammation caused by DAMPs released from the necrotic cells.

### [Citation List]

### [Non Patent Literature]

[Non Patent Literature 1]
   Zindel, J. and Kubes, P. (2020). DAMPs, PAMPs, and LAMPs in Immunity and Sterile Inflammation. Annu. Rev. Pathol. 15, 493-518.
[Non Patent Literature 2]
   Trejo-Gabriel-Galan, J.M. (2020). Stroke as a complication and prognostic factor of COVID-19. Neurologia 35, 318-322.
[Non Patent Literature 3]
   Oxley, T.J., Mocco, J., Majidi, S., Kellner, C.P., Shoirah, H., Singh, I.P., De Leacy, R.A., Shigematsu, T., Ladner, T.R.,
   Yaeger, K.A. et al. (2020). Large-Vessel Stroke as a Presenting Feature of Covid-19 in the Young. N. Engl. J. Med. 382, e60 (2020).
[Non Patent Literature 4]
   Tan, Y.K., Goh, C., Leow, A.S.T., Tambyah, P.A., Ang, A., Yap, E.S., Tu, T.M., Sharma, V.K., Yeo, L.L.L., Chan, B.P.L. et al. (2020). COVID-19 and ischemic stroke: a systematic review and meta-summary of the literature. J. Thromb. Thrombolysis 50, 587-595.
[Non Patent Literature 5]
   Gulke, E., Gelderblom, M. and Magnus, T. (2018). Danger signals in stroke and their role on microglia activation after ischemia. Ther Adv. Neurol. Disord. 11, 1756286418774254.
[Non Patent Literature 6]
   Chamorro, A., Dirnagl, U., Urra, X. and Planas, A.M. (2016). Neuroprotection in acute stroke: targeting excitotoxicity, oxidative and nitrosativestress, and inflammation. Lancet Neurol. 15, 869-881.
[Non Patent Literature 7]
   Land, W.G. (2020). Use of DAMPs and SAMPs as Therapeutic Targets or Therapeutics: A Note of Caution. Mol. Diagn. Ther. 24, 251-262.
[Non Patent Literature 8]
   Richard, S., Lapierre, V., Girerd, N., Bonnerot, M., Burkhard, P.R., Lagerstedt, L., Bracard, S., Debouverie, M., Turck, N. and Sanchez, J.-C. (2016). Diagnostic performance of peroxiredoxin 1 to determine time-of-onset of acute cerebral infarction. Sci. Rep. 6, 38300.
[Non Patent Literature 9]
   Shichita, S., Ito, M., Morita, R., Komai, K., Noguchi, Y., Ooboshi, H., Koshida, Y., Takahashi, S., Kodama, T. & Yoshimura, A. (2017). MAFB prevents excess inflammation after ischemic stroke by accelerating clearance of damage signals through MSR1. Nat Med. 23, 723-732.
[Non Patent Literature 10]
   Arai, S.,. Kitada, K., Yamazaki, T., Takai, R., Zhang, X., Tsugawa, Y., Sugisawa, R., Matsumoto, A., Mori, M., Yoshihara, Y., et al. (2016). Apoptosis inhibitor of macrophage protein enhances intraluminal debris clearance and ameliorates acute kidney injury in mice. Nat. Med. 22, 183-193.
[Non Patent Literature 11]
   Tomita, T., Arai, S., Kitada, K., Mizuno, M., Suzuki, Y., Sakata, F., Nakano, D., Hiramoto, E., Takei, Y., Maruyama, S. et al. (2017). Apoptosis inhibitor of macrophage ameliorates fungus-induced peritoneal injury model in mice. Sci. Rep. 7, 6450.
[Non Patent Literature 12]
   Arai, S. and Miyazaki, T. (2018). A scavenging system against internal pathogens promoted by the circulating protein apoptosis inhibitor of macrophage (AIM). Semin. Immunopathol. 40, 567-575.

### [Summary of Invention]

### [Technical Problem]

The present invention aims to provide a novel therapeutic agent and a novel treatment method for ischemic diseases including cerebral infarction.

### [Solution to Problem]

The present inventors have conducted intensive studies of the above-mentioned problems and found that (1) AIM binds to various DAMPs (e.g., PRDX1 - 6, HMGB1, S100A8, S100A8/9, S100A9, HSP70, etc.) in two coupling schemes, (2) binding of AIM to DAMPs neutralizes the biological activity of DAMPs, (3) binding of AIM to DAMPs results in efficient phagocytosis and removal of DAMPs by microglia and macrophages that have infiltrated into the infarct area, (4) as a result of (2) and (3), AIM suppresses the spread of sterile inflammation, and (5) AIM enhances phagocytic removal of dead cell debris in cerebral infarct region and suppresses the spread of sterile inflammation. Based on such findings, they have conducted further studies and completed the present invention.

Accordingly, the present invention provides the following.

[1] An agent for the treatment of an ischemic disease, comprising an apoptosis inhibitor of macrophage (AIM), an AIM fragment having a biological activity of AIM, or a nucleic acid encoding the AIM or AIM fragment.
[2] The agent of [1], wherein the ischemic disease is selected from the group consisting of cerebral infarction, myocardial infarction, limb ischemia, pulmonary infarction, splenic infarction, intestinal infarction, and Buerger's disease.
[3] An agent for the suppression of sterile inflammation mediated by damage-associated molecular patterns (DAMPs), comprising an apoptosis inhibitor of macrophage (AIM), an AIM fragment having a biological activity of AIM, or a nucleic acid encoding the AIM or AIM fragment.
[4] The agent of [3], wherein the DAMP is at least one selected from the group consisting of PRDX1, PRDX2, PRDX3, PRDX4, PRDX5, PRDX6, HMGB1, S100A8, S100A8/9, S100A9, and HSP70.
[5] A method for treating an ischemic disease, comprising administering an apoptosis inhibitor of macrophage (AIM), an AIM fragment having a biological activity of AIM, or a nucleic acid encoding the AIM or AIM fragment to a subject affected with the ischemic disease.
[6] The method of [5], wherein the ischemic disease is selected from the group consisting of cerebral infarction, myocardial infarction, limb ischemia, pulmonary infarction, splenic infarction, intestinal infarction, and Buerger's disease.
[7] A method for suppressing sterile inflammation mediated by damage-associated molecular patterns (DAMPs), comprising administering an apoptosis inhibitor of macrophage (AIM), an AIM fragment having a biological activity of AIM, or a nucleic acid encoding the AIM or AIM fragment to a subject affected with the sterile inflammation mediated by DAMPs.
[8] The method of [7], wherein the DAMP is at least one selected from the group consisting of PRDX1, PRDX2, PRDX3, PRDX4, PRDX5, PRDX6, HMGB1, S100A8, S100A8/9, S100A9, and HSP70.

### [Advantageous Effects of Invention]

According to the present invention, it is possible to efficiently remove dead cell debris in the infarcted region in the subject while simultaneously suppressing sterile inflammation. Therefore, the present invention is capable of treating ischemic diseases such as cerebral infarction, myocardial infarction, limb ischemia, pulmonary infarction, splenic infarction, intestinal infarction, and Buerger's disease.

### [Brief Description of Drawings]

[Fig. 1]

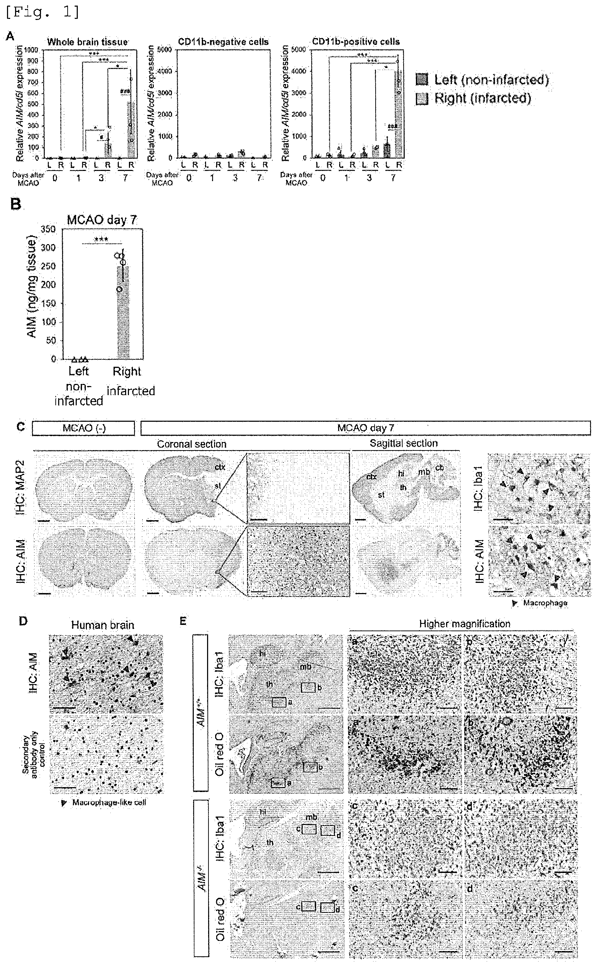

   Fig. 1 shows AIM expression in the infarcted brain. (A) Quantitative polymerase chain reaction (QPCR) analysis of AIM (cd5l) mRNA levels in the brain using RNA isolated from whole tissue or dissociated cells by CD11b (macrophage/myeloid marker) expression from wild-type mice on days 1, 3 and 7 after Middle Cerebral Artery Occlusion(MCAO). L: left lobe, non-infarcted; R: right lobe, infarcted. Relative values to that of whole tissue before MCAO (Pre) are shown. Bars: standard deviation of the mean. Statistics: multi-way ANOVA followed by Bonferroni's post hoc test. p: *< 0.05, ** < 0.01. *** < 0.001 (between the values at different days in the left or right lobe); #< 0.05, ### < 0.001 (between the values in the left and right lobe at the same day). n = 3. (B) The amount of AIM protein in the brain was analyzed by ELISA using lysates from the infarcted (right lobe; R) and non-infarcted (left lobe; L) brain. The values are presented as µg AIM/mg brain. n = 4. (C) Immunohistochemistry (IHC) analysis of AIM in brain specimens from day 7 post-MCAO wild-type mice. The infarcted area, which is negative for Microtubule-associated protein 2(MAP2), was stained for AIM. A part of striatum (red square) is shown at a higher magnification for AIM and MAP2 (3^{rd} lane from left, squared by red line). The right panel shows the serial section stained for Iba1 and AIM presented at a high magnification. Iba1-positive macrophages are strongly positive for AIM. Abbreviations: ctx: cortex, st: striatum, hi: hypothalamus, th: thalamus, mb: mid brain, cb: cerebrum. Bar: 1mm. The bar in the highly expanded panel in the coronal section indicates 100 mm, while the bar in the rightmost panel of IHC:Iba1 and IHC:AIM indicates 5 mm. (D) A human brain specimen from an infarcted region was stained for AIM. A control staining with only secondary antibody is also presented (lower panel). Note that whole area was MAP2-negative (data not presented). The strong staining for AIM indicates AIM production in macrophages (determined by their shape; indicated by arrows), in accordance with the QPCR data shown in (A). bars: 50 mm. (E) Serial frozen sections from wild-type and AIM ^{-/-} brains on day 7 post-MCAO were stained for oil-red O and Iba1. Many Iba1-positive macrophages at the infarcted corpus striatum region are positive for oil-red-O (red signals) in wild-type mice, whereas they are largely negative for oil-red-O in AIM^{-/-} mice. The bars in the four panels in the left column indicate 1 mm, and the bars in the eight panels in the center and right columns indicate 100 mm.
[Fig. 2]

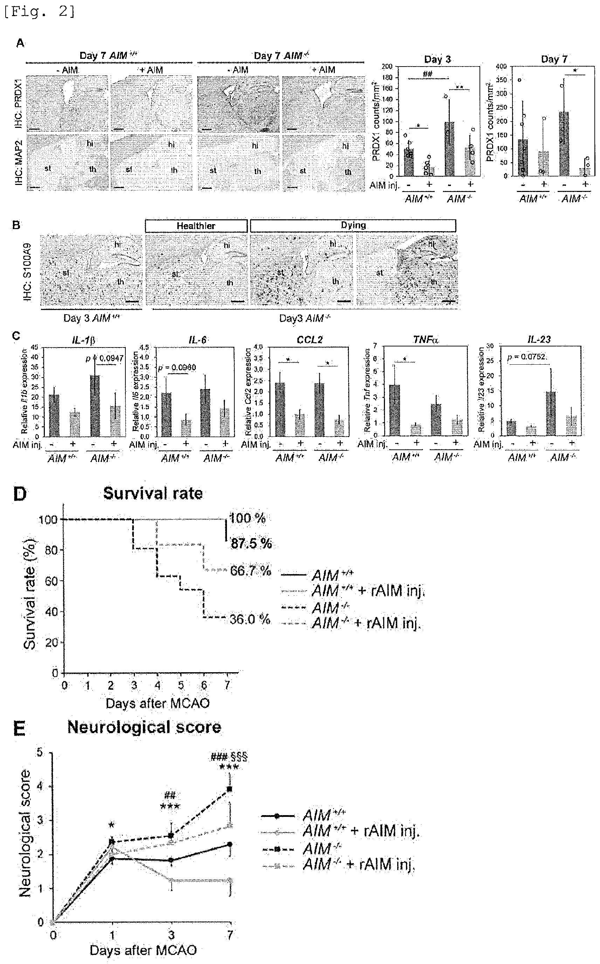

   Fig. 2 shows that AIM decreases DAMP levels and suppresses inflammation in the infarcted area, thereby improving post-stroke prognosis. (A) The brain specimens from wild-type (AIM^{+/+}) and AIM^{-/-} mice with or without rAIM injection (0.5 mg daily from day 1 after MCAO) on days 3 and 7 after MCAO were stained for PRDX1, HMGB1, or S100A9, as well as with hematoxylin to identify nuclei. Representative photos of PRDX1 (upper panels) and MAP2 (lower panels) staining on day 7 post-MCAO are presented. Bars: 500 mm. The number of positive signals for PRDX1 (brown signals) within the MAP2-negative region that were not co-localized with the hematoxylin signals was counted and normalized by the area (mm²) of MAP2-negative region. n = 3-8. Statistics: multi-way ANOVA followed by Bonferroni's post hoc test. p: * < 0.05; ** < 0.01 (comparison between with and without AIM injection). ## < 0.01 (AIM^{+/+} v. s. AIM^{-/-}). Abbreviations: st: striatum, hi: hypothalamus, th: thalamus. (B) S100A9 staining on day 3 in two dying and a healthier AIM^{-/-} mice, as well as a wild-type mouse. The brains from dying AIM^{-/-} mice showed a markedly larger number of intense signals than the other healthier AIM^{-/-} mice or wild-type mice on day 3. Abbreviations: st: striatum, hi: hypothalamus, th: thalamus. Note that the reduction of blood flow in the middle cerebral artery by MCAO was comparable in mice, confirming that the severity of MCAO was not different among the tested mice. Bars: 500 mm. (C) QPCR analysis of the mRNA levels of inflammatory cytokines using RNA from CD11⁺ macrophages/myeloid cells isolated from the infarcted brain in wild-type and AIM^{-/-} with or without rAIM injection (0.5 mg daily from day 1 after MCAO) on days 4 after MCAO. Relative values to those in CD11⁺ cells are presented. n = 4-6 each. The average values ± s. e. m. are presented. Statistics: multi-way ANOVA followed by Bonferroni's post hoc test. *p < 0.05. (D) Survival of wild-type, AIM^{-/-}, rAIM (0.5 mg)-injected AIM^{-/-}, and rAIM (0.1 mg)-injected wild-type mice after MCAO. n = 38 (wild-type), 21 (AIM^{-/-}), 9 (wild-type + rAIM injection), 12 (AIM^{-/-}+ rAIM injection). rAIM was injected intravenously daily from day 1. Statistical significance of the survival during the 7 days after MCAO was assessed using the generalized Wilcoxon test. p = 0.0000347 in wild-type mice vs. AIM^{-/-} mice; 0.0780 in AIM^{-/-} mice with rAIM vs AIM^{-/-} mice without rAIM injection. (E) Neurologic score in the four groups of mice as in (D). n = 17 (wild-type), 11 (AIM^{-/-}), 9 (wild-type + rAIM injection), 6 (AIM^{-/-}+ rAIM injection). Mean values ± s. e. m. are presented. Statistics: multi-way ANOVA followed by Bonferroni's post hoc test. p: * < 0.05; *** < 0.001 (wild-type vs. AIM^{-/-}), ## < 0.01; ### < 0.001 (wild-type vs. wild-type + rAIM injection), §§§ < 0.001 (AIM^{-/-} vs. AIM^{-/-}+ rAIM injection).
[Fig. 3]

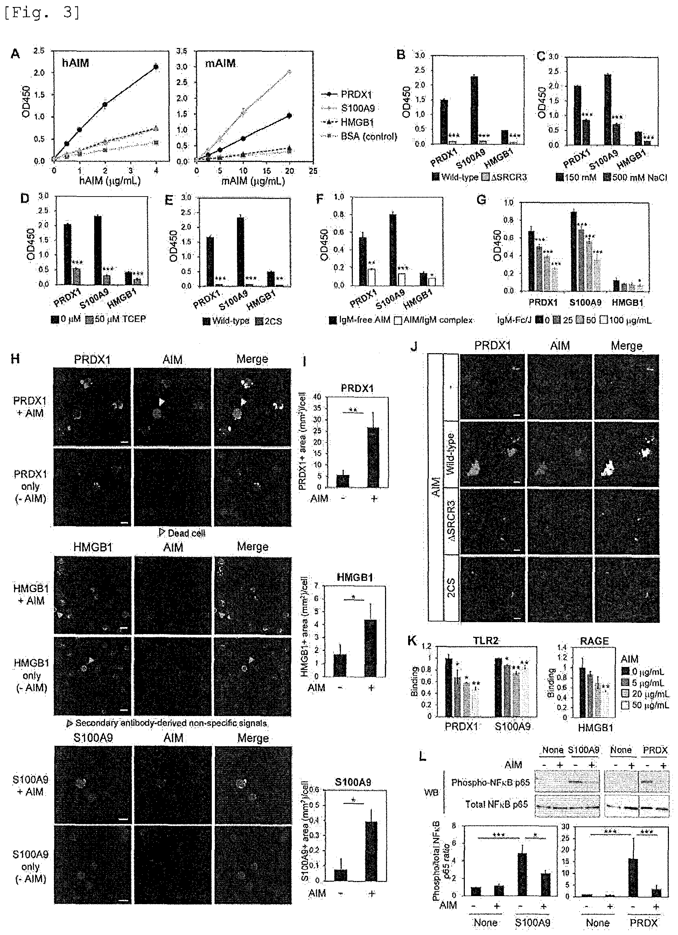

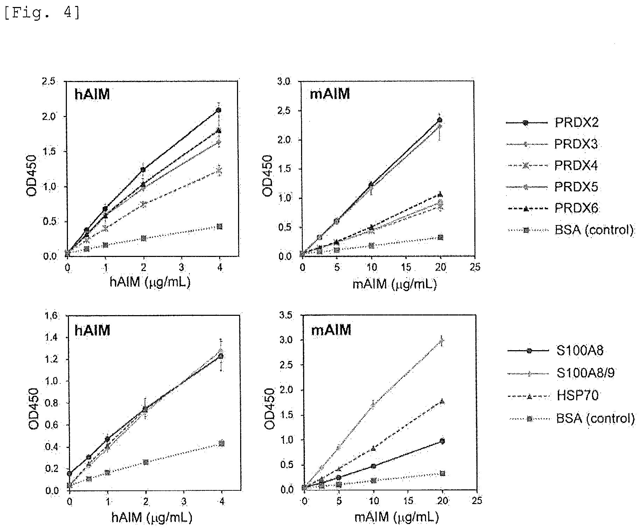

   Fig. 3 shows that AIM binds to DAMPs, thereby enhancing the phagocytic removal of DAMPs and interfering with the binding of DAMPs to pattern recognition receptors. (A) In vitro binding assay of AIM to various DAMPs. The binding abilities of different concentrations of human or mouse AIM to PRDX1, HMGB1, S100A9, and bovine serum albumin (BSA) as a control (coated on the plate at 2 µg/mL) are presented. The assay was performed in triplicate and the average values ± s. d. are presented. The results of AIM binding to DAMPs are presented in Figure 4. (B) The binding abilities of mouse ΔSRCR3 mutant or (C) wild-type mouse AIM (20 µg/mL each) in the presence of 150 mM (physiological) or 500 mM (excess) of NaCl to PRDX1, HMGB1, and S100A9. In the presence of excess NaCl, binding was largely diminished. (D) The binding abilities of mouse wild-type AIM (20 µg/mL) in the presence of 50 µM TCEP or (E) mouse 2CS mutant, to PRDX1, HMGB1, and S100A9. The similar result was obtained in human AIM (data not shown). (F) The binding ability of recombinant mouse AIM/IgM-Fc pentamer complexes to DAMPs performed as in (A). Binding was diminished. (G) Binding of mouse AIM to DAMPs was impaired by mouse IgM-Fc pentamer in a dose-dependent manner. In (B)-(G), the assay was performed in triplicate and the average values are presented. Statistics: Welch's t-test (B)-(F), one-way ANOVA followed by Bonferroni's post hoc test p: * < 0.05, ** <0.01, *** < 0.001 (vs. control). (H) Peritoneal macrophages isolated from AIM^{-/-}mice were challenged with PRDX1, HMGB1, and S100A9 on a chamber slide in the presence or absence of rAIM. After incubation for 10 minutes, the cells were fixed and stained for each DAMP (green), AIM (red), and nuclei (blue), and analyzed under a florescence confocal microscope. White areas indicate colocalization of DAMPs and AIM. Attachment of rAIM to some dead cells was also observed (yellow arrows). Non-specific signals (background) derived by secondary antibody developed in HMGB1 staining (orange arrows). The volume of each PRDX1, HMGB1, or S100A9 present at the cell surface and intracellulary was determined using software Halo and presented in the graph. Values are averages of the results from 3 experiments (n = 4-13). Statistics: Welch's t-test. p: * < 0.05, ** <0.01. (J) Induction of PRDX1 aggregation by AIM (wild-type), ΔSRCR3 mutant, and 2CS mutant. (K) Binding of PRDX1 and S100A9 to TLR2 and HMGB1 to RAGE in the presence or absence of various concentrations of rAIM. The presence of rAIM reduced the ability of PRDX1 and S100A9 to bind to TLR2 and HMGB1 to RAGE in a dose-dependent manner. The assay was performed in triplicate and the average values are presented. Statistics: one-way ANOVA followed by Bonferroni's *post hoc* test. *p*: * < 0.05, ** <0.01. (L) RAW264.1 mouse macrophage cells were incubated with mouse PRDX or S100A9 for 30 min at 37°C in the presence or absence of rAIM. Thereafter, the cells were harvested, and the cell lysates were analyzed for phosphorylation (indicating NFKB activation) by immunoblotting. The intensity of the signals was quantified and presented in the graphs. Values represent intensity of the phosphorylated NFKB p65 relative to that of total NFKB p65. The presence of rAIM decreased NFκB phosphorylation. Statistics: two-way ANOVA followed by Bonferroni's post hoc test. p: * < 0.05, *** < 0.001.
[Fig. 4]
   Fig 4 shows the binding of AIM to various DAMPs. The binding abilities of different concentrations of human or mouse AIM to PRDXs, S100A8, S100A8/9, HSP70, and bovine serum albumin (BSA) as a control (coated on the plate at 2 µg/mL) are presented. The assay was performed in triplicate and the average values ± s. d. are presented.
[Fig. 5]

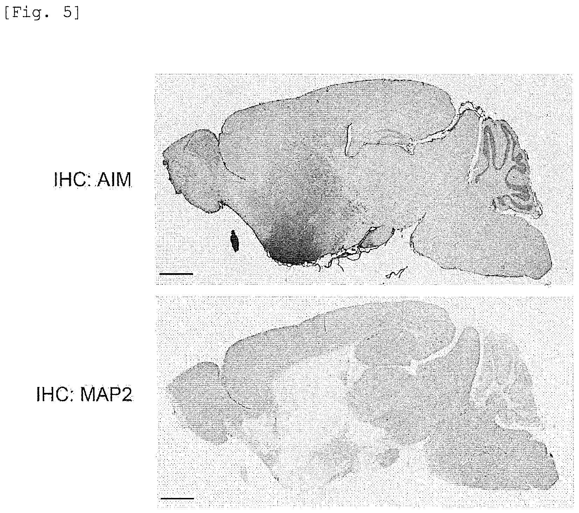

   Fig 5 shows that rAIM passed through the Blood-Brain-Barrier. AIM^{-/-} mice challenged with MCAO were injected with 0.5 mg of rAIM intravenously on day 3 after MCAO, and sacrificed 1h after rAIM injection. The sagittal sections of the infarcted side of the brain were stained for AIM (upper) and MAP2 (lower). MAP2-negative infarcted area is stained for AIM. Scale bars: 1 mm.

### [Description of Embodiments]

The present invention is described in detail in the following.

### 1. Agent for the treatment of an ischemic disease

The present invention provides an agent for the treatment of an ischemic disease that contains an apoptosis inhibitor of macrophage (AIM), an AIM fragment having the biological activity of AIM, or a nucleic acid encoding the AIM or AIM fragment (hereinafter sometimes to be referred to as "the agent for the treatment of the present invention").

Ischemic diseases are diseases in which organs become ischemic due to obstruction or stenosis of arteries that provide nutrition to the organs, and tissues become necrotic or dysfunctional due to the shortage of oxygen or nutrients. Examples of the ischemic diseases treated with the agent for the treatment of the present invention include, but are not limited to, cerebral infarction, myocardial infarction, limb ischemia, pulmonary infarction, splenic infarction, intestinal infarction, Buerger's disease, and the like. In one preferred embodiment, the ischemic disease could be cerebral infarction.

Cerebral infarction is classified based on the mechanism of onset. Cerebral infarction that occurs when blood flow is obstructed as a result of arteriosclerosis that has progressed in relatively large blood vessels in the neck and brain is called "atherothrombotic brain infarction". Cerebral infarction that occurs due to blockage of small blood vessels deep in the brain is called "lacunar infarction". Cerebral infarction that occurs when a blood vessel in the brain is blocked by a blood clot formed in the heart and transported to the brain is called "cardiogenic cerebral embolism". The agent for the treatment of the present invention is applicable to the treatment of any type of cerebral infarction.

AIM to be used in the present invention is a protein containing an amino acid sequence that is the same or substantially the same as the amino acid sequence shown in SEQ ID NO: 1 (amino acid sequence of human-derived AIM protein). AIM may be, for example, a protein isolated and purified from macrophage, which is immunocyte of warm-blooded animals (e.g., human, mouse, rat, rabbit, sheep, swine, bovine, horse, cat, dog, monkey, chimpanzee, bird, and the like). It may also be a protein chemically synthesized or biochemically synthesized in a cell-free translation system. Alternatively, the protein may be a recombinant protein produced from a transformant incorporating a nucleic acid comprising a base sequence that encodes the above-described amino acid sequence. The species of organism to be the origin of the AIM to be used in the present invention may be preferably the same as the species of the subject suffering from a neurodegenerative disease. For example, when the application target of the agent for the treatment of the present invention is a human, human AIM is preferably used.

Substantially the same amino acid sequence as the amino acid sequence shown in SEQ ID NO:1 refers to an amino acid sequence having an identity or similarity of about 60% or more, preferably about 70% or more, further preferably about 80% or more, particularly preferably about 90% or more, most preferably about 95% or more, to the amino acid sequence shown in SEQ ID NO:1, and the like. As used herein, the "identity" means the proportion (%) of the same amino acids and similar amino acid residues to the overlapping total amino acid residues in the optimal alignment (preferably, the algorithm can consider, for the optimal alignment, introduction of a gap into one or both of the sequences), when two amino acid sequences are aligned using mathematical algorithm known in the technical field. The "similarity" means the ratio (%) of the number of positions where the same or similar amino acid residues are present in two amino acid sequences when they are aligned to the total number of amino acid residues. The "similar amino acid" means amino acids similar in physicochemical properties and, for example, amino acids classified into the same group such as aromatic amino acid (Phe, Trp, Tyr), aliphatic amino acid (Ala, Leu, Ile, Val), polar amino acid (Gin, Asn), basic amino acid (Lys, Arg, His), acidic amino acid (Glu, Asp), amino acid having hydroxyl group (Ser, Thr), amino acid with small side chain (Gly, Ala, Ser, Thr, Met) and the like can be mentioned. Substitution with such similar amino acids is predicted to cause no change in the phenotype of the protein (i.e., conservative amino acid substitution). Specific examples of the conservative amino acid substitution are well known in the art and described in various documents (see, for example, Bowie et al., Science, 247:1306-1310(1990)).

The identity or similarity of the amino acid sequence in the present specification can be calculated using the identity or similarity calculation algorithm NCBI BLAST (National Center for Biotechnology Information Basic Local Alignment Search Tool) under the following conditions (expectancy=10; gap; matrix =BLOSUM62; filtering=OFF). Examples of other algorithm to determine the identity or similarity of amino acid sequence include the algorithm described in Karlin et al., Proc. Natl. Acad. Sci. USA, 90:5873-5877(1993) [the algorithm is incorporated in NBLAST and XBLAST program (version 2.0) (Altschul et al., Nucleic Acids Res., 25:3389-3402(1997))], the algorithm described in Needleman et al., J. Mol. Biol., 48:444-453(1970) [the algorithm is incorporated in GAP program in GCG software package], the algorithm described in Myers and Miller, CABIOS, 4:11-17(1988) [the algorithm is incorporated in ALIGN program (version 2.0) which is a part of the CGC sequence alignment software package], the algorithm described in Pearson et al., Proc. Natl. Acad. Sci. USA, 85: 2444-2448(1988) [the algorithm is incorporated in FASTA program in the GCG software package] and the like, and they can also be preferably used in the same manner. More preferably, substantially the same amino acid sequence as the amino acid sequence shown in SEQ ID NO:1 is an amino acid sequence having an identity of about 60% or more, preferably about 70% or more, further preferably about 80% or more, particularly preferably about 90% or more, and most preferably about 95% or more, to the amino acid sequence shown in SEQ ID NO:1.

As a protein comprising substantially the same amino acid sequence as the amino acid sequence shown in SEQ ID NO:1, for example, a protein comprising substantially the same amino acid sequence as the aforementioned amino acid sequence shown in SEQ ID NO:1, and having substantially the same quality of activity as the biological activity of wild-type AIM can be mentioned. Examples of the biological activity of the wild-type AIM include endocytosis activity on macrophage (including microglia), activity to suppress apoptosis of macrophage, activity to maintain or promote arteriosclerosis, adipocyte differentiation suppressive activity, activity to melt lipid droplet of adipocyte, adipocyte reducing activity, CD36 binding activity, endocytosis activity to adipocyte, FAS binding activity, FAS function suppressive activity, antiobesity activity, activity to prevent or treat hepatic diseases (fatty liver, NASH, cirrhosis, liver cancer), activity to prevent or treat renal diseases (acute renal failure, chronic nephritis, chronic renal failure, nephrotic syndrome, diabetic nephropathy, nephrosclerosis, IgA nephropathy, hypertensive nephropathy, nephropathy with collagen disease or IgM nephropathy) and the like. In the present invention, particularly, an endocytosis activity on macrophage can be a preferable index. The activity can be confirmed using in vitro macrophage (or microglia) phagocytosis tests, though not limited thereto. In the present specification, the "substantially the same quality" means that the activity thereof is qualitatively (e.g., physiologically or pharmacologically) the same. Therefore, it is preferable that the aforementioned activities be equivalent to each other, but the quantitative factors of these activities, such as the extent of activity (e.g., about 0.1 to about 10 times, preferably about 0.5 to about 2 times) and the molecular weight of the protein, may be different. The aforementioned activities can be measured by a method known per se.

Examples of the AIM to be used in the present invention also include proteins comprising (1) an amino acid sequence having 1 or 2 or more (preferably about 1 to 100, preferably about 1 to 50, further preferably about 1 to 10, particularly preferably 1 to several (2, 3, 4 or 5)) amino acids deleted from the amino acid sequence shown in SEQ ID NO:1, (2) an amino acid sequence having 1 or 2 or more (preferably about 1 to 100, preferably about 1 to 50, further preferably about 1 to 10, particularly preferably 1 to several (2, 3, 4 or 5)) amino acids added to the amino acid sequence shown in SEQ ID NO:1, (3) an amino acid sequence having 1 or 2 or more (preferably about 1 to 50, preferably about 1 to 10, further preferably 1 to several (2, 3, 4 or 5)) amino acids inserted in the amino acid sequence shown in SEQ ID NO:1, (4) an amino acid sequence having 1 or 2 or more (preferably about 1 to 50, preferably about 1 to 10, further preferably 1 to several (2, 3, 4 or 5)) amino acids substituted by other amino acids in the amino acid sequence shown in SEQ ID NO:1, or (5) an amino acid sequence comprising a combination thereof. When an amino acid sequence has been inserted, deleted or substituted as described above, the position of the insertion, deletion or substitution is not particularly limited as long as a desired biological activity (e.g., endocytosis activity on macrophage (including microglia)) of the protein is maintained.

AIM in the present invention is preferably a human AIM protein having the amino acid sequence shown in SEQ ID NO:1 (GenBank Accession No.: AAD01446), or a homologue thereof in other mammals [for example, mouse homologue registered in the GenBank as Accession No.: AAD01445 and the like], more preferably, a human AIM protein consisting of the amino acid sequence shown in SEQ ID NO:1.

In the present specification, the protein and peptide are described according to conventional peptide lettering, and the left end is N-terminus (amino terminus), and the right end is C-terminus (carboxyl terminus). In AIM to be used in the present invention including a protein comprising the amino acid sequence shown in SEQ ID NO:1, the C-terminus may be any of a carboxyl group (-COOH), carboxylate (-COO⁻), amide (-CONH₂) and ester (-COOR).

Here, as R in the ester, a C₁₋₆ alkyl group, for example, methyl, ethyl, n-propyl, isopropyl and n-butyl, a C₃₋₈ cycloalkyl group, for example, cyclopentyl and cyclohexyl, a C₆₋₁₂ aryl group, for example, phenyl and α-naphthyl, a phenyl-C₁₋₂ alkyl group, for example, benzyl and phenethyl, a C₇₋₁₄ aralkyl group, for example, an α-naphthyl-C₁₋₂ alkyl group, for example, α-naphthylmethyl, a pivaloyloxymethyl group; and the like can be used.

When the AIM to be used in the present invention has a carboxyl group (or carboxylate) at a position other than the C-terminus, a protein wherein the carboxyl group is amidated or esterified is also included in the protein of the present invention. In this case, as the ester, the above-described ester at the C terminus, and the like, for example, is used.

Furthermore, the AIM to be used in the present invention also includes a protein wherein the amino group of the N-terminal amino acid residue is protected by a protecting group (e.g., C₁₋₆ acyl groups such as C₁₋₆ alkanoyls such as formyl group and acetyl group, and the like); a protein wherein the glutamine residue that may be produced upon cleavage at the N terminus in vivo has been converted to pyroglutamic acid, a protein wherein a substituent (e.g., -OH, -SH, amino group, imidazole group, indol group, guanidino group and the like) on a side chain of an amino acid in the molecule is protected by an appropriate protecting group (e.g., C₁₋₆ acyl groups such as C₁₋₆ alkanoyl groups such as formyl group and acetyl group, and the like), a conjugated peptide such as what is called a glycopeptide having a sugar chain bound thereto, and the like.

In the present specification, the "AIM" is a concept including not only wild-type AIM but also mutants of these and the like having an activity substantially the same or improved than the biological activity of the wild-type AIM. Here, the "activity of substantially the same quality" is as defined above. In addition, the "activity of substantially the same quality" can be measured in the same manner as in the case of AIM.

One embodiment of the mutant of AIM includes, but is not limited to, the following.

The mutant human AIM of the present invention preferably contains the amino acid sequence of any one of the following (1b) to (5b).
(1b) an amino acid sequence wherein cysteine at amino acid number 191 of the amino acid sequence shown in SEQ ID NO: 1 is substituted with serine,
(2b) an amino acid sequence wherein cysteine at amino acid number 300 of the amino acid sequence shown in SEQ ID NO: 1 is substituted with serine,
(3b) an amino acid sequence wherein cysteine at amino acid number 191 of the amino acid sequence shown in SEQ ID NO: 1 is substituted with serine, and an amino acid sequence wherein cysteine at amino acid number 300 of the amino acid sequence shown in SEQ ID NO: 1 is substituted with serine,
(4b) an amino acid sequence substantially the same as the amino acid sequence of any one of (1b) to (3b), wherein cysteines present in the amino acid sequence of any one of (1b) to (3b) and the substituted serine remain,
(5b) an amino acid sequence further comprising deletion, addition, insertion or substitution of one to several amino acids or a combination thereof at a position(s) other than cysteines present in the amino acid sequence of any one of (1b) to (3b) and the substituted serine.

As an AIM mutant having a function the same as or improved than the function of the wild-type recombinant AIM, one disclosed in Patent Application No. JP2017-220733 or the like can be used.

As a component of the agent for the treatment of the present invention, not only AIM but also an AIM fragment having the biological activity of AIM can also be used. Whether or not the AIM fragment has the biological activity of wild-type AIM may be determined by the aforementioned method.

As one embodiment of an AIM fragment, since intact AIM protein contains 3 SRCR (Scavenger-Receptor Cysteine-Rich) domains containing a large amount of cysteine, each SRCR domain can be recited as an example of an AIM fragment having substantially the same quality of activity as the biological activity possessed by the wild-type AIM. To be specific, for example, of the amino acid sequence shown in SEQ ID NO:1, partial amino acid sequences respectively comprising SRCR1 domain (amino acid Nos. 24 - 125 of the amino acid sequence shown in SEQ ID NO:1), SRCR2 domain (amino acid Nos. 138 - 239 of the amino acid sequence shown in SEQ ID NO:1), and SRCR3 domain (amino acid Nos. 244 - 346 of the amino acid sequence shown in SEQ ID NO:1), partial amino acid sequence comprising any combination of SRCR domains and the like can be used as the AIM fragment. The size of the AIM fragment having substantially the same quality of activity as the biological activity possessed by the wild-type AIM is not particularly limited as long as it comprises the above-mentioned functional domain. The partial peptide preferably comprises not less than 50 partial amino acid sequences, more preferably not less than 100 partial amino acid sequences, further preferably not less than 200 partial amino acid sequences. The partial amino acid sequences may be a single consecutive partial amino acid sequence, or discontinuous plural partial amino acid sequences linked to each other.

In addition, the C-terminus of the AIM fragment to be used in the present invention may be any of a carboxyl group (-COOH), carboxylate (-COO⁻), amide (-CONH₂) and ester (-COOR). Here, examples of the R in ester include those similar to the examples recited above for AIM. When the partial peptide of the present invention has a carboxyl group (or carboxylate) at a position other than the C-terminus, the carboxyl group may be amidated or esterified, which is also encompassed in the partial peptide of the present invention. As the ester in this case, for example, those similar to the ester at the C-terminus and the like are used.

Furthermore, the AIM fragment to be used in the present invention includes, in the same manner as in the above-mentioned AIM, the amino group of the N-terminal amino acid residue may be protected with a protecting group, the glutamine residue at the N-terminus may be converted to pyroglutamic acid, a substituent on the side chain of the amino acid in a molecule may be protected with a suitable protecting group, or the partial peptide may be a composite peptide wherein a sugar chain is bonded (so-called glycopeptide and the like), and the like.

The AIM (including AIM fragment) to be used in the present invention may be in the form of a salt. For example, salts with physiologically acceptable acid (e.g., inorganic acid, organic acid), base (e.g., alkali metal salt) and the like are used, and a physiologically acceptable acid addition salt is particularly preferable. Examples of such salt to be used include salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

AIM can be produced from a macrophage of the aforementioned mammals by a protein purification method known per se. To be specific, AIM or a salt thereof can be prepared by homogenizing mammalian macrophage, removing cell debris by low-speed centrifugation, centrifuging the supernatant at a high speed to precipitate a cellular membrane-comprising fraction, and subjecting the supernatant to chromatography such as reversed-phase chromatography, ion exchange chromatography, affinity chromatography and the like, and the like.

AIM (including AIM fragments) can also be produced according to a publicly known method of peptide synthesis. The method of peptide synthesis may be any of, for example, a solid phase synthesis process and a liquid phase synthesis process. A desired protein can be produced by condensing a partial peptide or amino acid capable of constituting AIM with the remaining portion, and removing any protecting group the resultant product may have.

Here, the condensation and the protecting group removal are conducted in accordance with methods known per se, for example, the methods indicated in (1) and (2) below:
(1) M. Bodanszky and M.A. Ondetti: Peptide Synthesis, Interscience Publishers, New York (1966)
(2) Schroeder and Luebke: The Peptide, Academic Press, New York (1965).

AIM thus obtained can be purified or isolated by a known method of purification. Here, as examples of the method of purification, solvent extraction, distillation, column chromatography, liquid chromatography, recrystallization, combinations thereof and the like can be mentioned.

When thus obtained AIM is in a free form, the free form can be converted into a suitable salt form by a known method or a method based thereon, and on the other hand, when the AIM is obtained in the form of a salt, it can be converted into the free form or in the form of a different salt by a known method or a method based thereon.

Furthermore, AIM can also be produced by culturing a transformant comprising a nucleic acid encoding the same, and separating and purifying AIM from the obtained culture. The nucleic acid encoding AIM or AIM fragment may be DNA or RNA, or DNA/RNA chimera, preferably DNA. Additionally, the nucleic acid may be double-stranded or single-stranded. In the case of a double-stranded nucleic acid, it may be a double-stranded DNA, a double-stranded RNA, or a DNA:RNA hybrid. In the case of a single strand, it may be a sense strand (that is, coding strand), or an antisense strand (that is, non-coding strand).

Examples of the DNA encoding AIM (including AIM fragments) include genomic DNA, cDNA derived from macrophage of warm-blooded animal (e.g., human, bovine, monkey, horse, swine, sheep, goat, dog, cat, guinea pig, rat, mouse, rabbit, hamster, chicken and the like), synthetic DNA and the like. Genomic DNA encoding AIM or an AIM fragment can be directly amplified by Polymerase Chain Reaction (hereinafter to be abbreviated as "PCR method") by using, as a template, a genomic DNA fraction prepared from any cell of the aforementioned animals [for example, hepatocyte, splenocyte, nerve cell, glial cell, pancreatic β cell, myelocyte, mesangial cell, Langerhans' cell, epidermal cell, epithelial cell, goblet cell, endothelial cell, smooth muscle cell, fibroblast, fibrocyte, myocyte, adipocyte, immunocyte (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, hepatocyte or interstitial cell, or corresponding progenitor cell, stem cell or cancer cell thereof, and the like], or any tissue where such cells are present [for example, brain or any portion of the brain (e.g., olfactory bulb, amygdaloid nucleus, basal ganglia, hippocampus, thalamus, hypothalamus, subthalamic nucleus, cerebral cortex, medulla oblongata, cerebellum), spinal cord, hypophysis, stomach, pancreas, kidney, liver, gonad, thyroid, gall-bladder, bone marrow, adrenal gland, skin, lung, gastrointestinal tract (e.g., large intestine, small intestine), blood vessel, heart, thymus, spleen, submandibular gland, peripheral blood, prostate, testicle, ovary, placenta, uterus, bone, joint, adipose tissue (e.g., brown adipose tissue, white adipose tissue), skeletal muscle and the like], and cDNA encoding AIM or an AIM fragment can also be directly amplified by PCR method and Reverse Transcriptase-PCR (hereinafter to be abbreviated as "RT-PCR method") by using, as a template, a total RNA or mRNA fraction prepared from macrophage, respectively. Alternatively, the genomic DNA and cDNA encoding AIM or a peptide fragment thereof can also be cloned by colony or plaque hybridization method or PCR method and the like from a genomic DNA library and cDNA library prepared by inserting the above-mentioned genomic DNA and total RNA or a fragment of mRNA into a suitable vector. The vector used for the library may be any of a bacteriophage, a plasmid, a cosmid, a phagemid and the like.

Examples of the nucleic acid encoding AIM include a nucleic acid comprising the same or substantially the same base sequence as the base sequence shown in SEQ ID NO: 2 and the like. As the nucleic acid comprising the same or substantially the same base sequence as the base sequence shown in SEQ ID NO: 2, for example, a nucleic acid comprising a base sequence having an identity or similarity of not less than about 60%, preferably not less than about 70%, more preferably not less than about 80%, particularly preferably not less than about 90%, with the base sequence shown in SEQ ID NO: 2, and encoding a protein having an activity of substantially the same quality as the aforementioned AIM and the like can be used. In one embodiment, the nucleic acid comprising the same or substantially the same base sequence as the base sequence shown in SEQ ID NO: 2 is a nucleic acid comprising a base sequence having a homology of not less than about 60%, preferably not less than about 70%, more preferably not less than about 80%, particularly preferably not less than about 90%, with the base sequence shown in SEQ ID NO: 2, and encoding a protein having the same or substantially the same quality of activity as the aforementioned AIM. The nucleic acid encoding AIM also includes a nucleic acid sequence subjected to codon optimization for the purpose of increasing expression efficiency in an organism to be the application target.

The identity or similarity of the base sequence in the present specification can be calculated under the following conditions (an expectation value = 10; gaps are allowed; filtering = ON, match score=1; mismatch score=-3) using an identity or similarity scoring algorithm NCBI BLAST (National Center for Biotechnology Information Basic Local Alignment Search Tool). As other algorithm for determining the identity or similarity of the base sequence, for example, the above-mentioned homology calculation algorithm for amino acid sequences can be similarly preferably recited as the example.

The nucleic acid encoding AIM is preferably a nucleic acid comprising a base sequence encoding human AIM protein shown by the base sequence shown in SEQ ID NO: 2 (GenBank accession No: AF011429), or a homologue thereof in other mammal [for example, mouse homologue registered in GenBank as accession No: AF011428 and the like].

As the component of the agent for the treatment of the present invention, a nucleic acid encoding AIM, or an AIM fragment having a biological activity of AIM can also be used.

The nucleic acid encoding AIM or AIM fragment to be used in the present invention may be any as long as it comprises a base sequence encoding a peptide comprising the same or substantially the same amino acid sequence as a part of the amino acid sequence shown in SEQ ID NO:1. Specifically, as a nucleic acid encoding the AIM fragment, (1) a nucleic acid comprising a partial base sequence of the base sequence shown in SEQ ID NO: 2, or (2) a nucleic acid comprising a base sequence having an identity or similarity of not less than about 60%, preferably not less than about 70%, more preferably not less than about 80%, particularly preferably not less than about 90%, with a nucleic acid comprising a partial base sequence of the base sequence shown in SEQ ID NO: 2, and encoding a protein having an activity of substantially the same quality as the aforementioned AIM and the like are used.

The nucleic acid encoding AIM or AIM fragment can be cloned by amplifying same using a synthesized DNA primer having a part of a base sequence encoding the AIM or AIM fragment by PCR method, or by conducting hybridization of a DNA incorporated into a suitable expression vector with a labeled DNA fragment or synthetic DNA encoding a part or whole region of AIM. Hybridization can be conducted according to a method known per se or a method based thereon, for example, a method described in Molecular Cloning, 2nd edition (J. Sambrook et al., Cold Spring Harbor Lab. Press, 1989) and the like. When a commercially available library is used, hybridization can be conducted according to the method described in the instruction manual attached thereto. Hybridization can preferably be conducted under highly stringent conditions.

As examples of the highly stringent conditions, conditions of a hybridization reaction in 6×SSC (sodium chloride/sodium citrate) at 45°C followed by washing in 0.2×SSC/0.1% SDS at 65°C once or more and the like can be mentioned. Those skilled in the art are able to easily obtain desired stringency by changing the salt concentration of the hybridization solution, hybridization reaction temperature, probe concentration, probe length, the number of mismatches, hybridization reaction time, the salt concentration of the washing solution, washing temperature and the like as appropriate. When a commercially available library is used, hybridization can be conducted according to the method described in the instruction manual attached to the library.

The nucleic acid encoding AIM or AIM fragment may also be functionally linked to an expression vector or the like having a promoter that is specifically expressed in the brain. By delivering an expression vector containing a nucleic acid encoding AIM or AIM fragment into the brain, the AIM or AIM fragment can be specifically expressed in the brain. Examples of the brain specific promoter include, but are not limited to, SCG10, GFAP promoter, synapsin 1 promoter, tubulin α1 promoter, calcium/calmodulin-dependent protein kinase II promoter, neuron-specific enolase promoter, PDGF (platelet-derived growth factor beta)-β chain promoter and the like.

In one preferable embodiment, a nucleic acid encoding AIM or AIM fragment may be mounted on a viral vector. Preferable examples of the viral vector include, but are not limited to, adeno-associated virus, adenovirus, lentivirus, and Sendai virus. In consideration of the use in gene therapy, adeno-associated virus is preferable because it can express transgene for a long period of time, is highly safe because it is derived from a non-pathogenic virus and the like. In addition, the serotype of the adeno-associated virus is not particularly limited as long as the desired effect of the present invention can be obtained, and any of serotypes 1, 2, 3, 4, 5, 6, 7, 8, 9, and 10 may also be used. When cerebral infarction is set as a therapeutic target in one embodiment of the present invention, particularly considering the high expression efficiency in neural tissues, serotype 1, 2, 5, 9, or 10 is preferred (see WO 2005/033321 for various serotypes of AAV). Furthermore, AAV5 is more preferred from the aspect of high expression efficiency, and serotype 9 (AAV9) is more preferred from the aspect of the property of efficient permeation through the blood vessel brain barrier (Iwata N et al, Sci Rep. 2013; 3:1472). The viral vector to be used in the present invention also includes variants thereof. As a variant of a viral vector, a modified capsid and the like are known. Particularly, examples of the AAV variant include, but are not limited to, those disclosed in WO 2012/057363 and the like.

When a nucleic acid encoding AIM or AIM fragment is mounted into a viral vector, for the purpose of specifically expressing AIM or AIM fragment in the target brain, it is preferable to use a brain-specific promoter as a promoter that controls the expression of the nucleic acid encoding AIM or AIM fragment. Examples of such brain-specific promoter include, but are not limited to, SCG10, GFAP promoter, synapsin 1 promoter, tubulin α1 promoter, calcium/calmodulin-dependent protein kinase II promoter, neuron-specific enolase promoter, PDGF (platelet-derived growth factor beta)-β chain promoter and the like. In addition to the promoter, known sequences such as Poly A addition signal, Kozak consensus sequence, tag sequence, linker sequence, NLS and the like may also be mounted as appropriate into a viral vector together with the nucleic acid encoding AIM or AIM fragment according to the purpose.

A viral vector containing a nucleic acid encoding AIM or AIM fragment can be prepared by a known method. In brief, a plasmid vector for virus expression is prepared by inserting a nucleic acid encoding AIM or AIM fragment and, if necessary, a nucleic acid having a desired function (e.g., brain-specific promoter, etc.), this is transfected into an appropriate host cell to transiently produce a viral vector containing the polynucleotide of the present invention, and the viral vector is recovered.

For example, when AAV vector is prepared, a vector plasmid is created first in which the ITRs at both ends of a wild-type AAV genome sequence are left and, in the place of a DNA encoding the other Rep proteins and capsid proteins, a nucleic acid encoding AIM or AIM fragment is inserted. On the other hand, the DNAs encoding Rep proteins and capsid proteins, which is necessary for forming virus particles, are inserted into another plasmid. Furthermore, a plasmid containing genes (E1A, E1B, E2A, VA, and E4orf6) responsible for the adenovirus helper action necessary for the proliferation of AAV is prepared as an adenovirus helper plasmid. These three plasmids are co-transfected into a host cell, whereby recombinant AAV (i.e., AAV vector) is produced within the cell. As the host cell, it is preferable to use a cell (e.g., 293 cell, etc.) that can supply a part of the gene product(s) (proteins) of the gene(s) responsible for the aforementioned helper action, and when such a cell is used, it is not necessary to mount the gene encoding the protein(s) that can be supplied from the host cell on the aforementioned adenovirus helper plasmid. Since the produced AAV vector exists in the nucleus, the host cell is frozen and thawed and recovered, and separated and purified by density ultracentrifugation method using cesium chloride, column method, and the like, whereby the desired AAV vector is prepared.

When the agent for the treatment of the present invention is used to treat or prevent an ischemic disease of a subject, the route of administration thereof is not particularly limited as long as the delivery of the active ingredient AIM protein into the affected part is realized. Examples of the preferred administration route include, but are not limited to, intravenous administration, intraarterial administration, subcutaneous administration, intraperitoneal administration, and the like.

In one embodiment, when the active ingredient of the agent for the treatment of the present invention is an AIM protein or a functional fragment thereof (hereinafter sometimes to be referred to as "AIM protein, etc.") and the ischemic disease is cerebral infarction, it is known that AIM protein, etc. generally do not pass through the Blood-Brain Barrier (BBB). However, as demonstrated in the following Example, the BBB is temporarily and partially destroyed at the onset of cerebral infarction, and as a result, AIM protein, etc. can be delivered to the cerebral infarction area by intravenous administration. When BBB is functional, for example, the agent for the treatment of the present invention may be directly injected into the brain tissue by a method known per se, such as microinjection or the like, through a pinhole made in the cranial bone of the subject. Alternatively, the AIM protein can also be delivered into the brain by encapsulating the AIM protein in a liposome modified to ensure permeation through the Blood-Brain Barrier and administering the liposome to the subject by intravenous administration or the like. Also, even when the component contained in the agent for the treatment of the present invention is a nucleic acid encoding AIM protein, the nucleic acid encoding the AIM protein can be delivered into the brain by a method of directly introducing the agent for the treatment of the present invention from a pinhole made in the cranial bone of a subject, a method using the aforementioned liposome, or the like.

When the component of the agent for the treatment of the present invention is a viral vector mounting a nucleic acid encoding AIM, a pinhole is provided in the cranial bone of a subject, and the agent for the treatment of the present invention can be directly introduced into the brain tissue via the pinhole by a method known per se, such as microinjection. As mentioned above, use of AAV9 is highly preferable since AIM can be specifically expressed in the brain of the subject simply by injecting the agent for the treatment of the present invention into the circulating blood without providing a pinhole in the cranial bone of the subject. That is, in an embodiment using means capable of crossing the Blood-Brain Barrier such as liposome, AAV9 and the like, the agent for the treatment of the present invention is parenterally administered and can be administered by, for example, intravenous administration, intraarterial administration, subcutaneous administration, or intraperitoneal administration.

When the agent for the treatment of the present invention is formulated for parenteral administration, for example, it can be formulated as injection, suppository or the like. The injection may include the dosage forms of intravenous injection, subcutaneous injection, intradermal injection, intramuscular injection, drip injection, and the like. Such injection can be prepared according to a known method. Injection can be prepared, for example, by dissolving, suspending or emulsifying components such as AIM, nucleic acid encoding AIM and/or virus mounting a nucleic acid encoding AIM and the like in a sterile aqueous solution or an oily solution generally used for injection. As the aqueous solution for injection, for example, saline, isotonic solution containing glucose and other auxiliary agents, and the like are used, which may be used in combination with a suitable solubilizing agent, for example, alcohol (e.g. ethanol), polyalcohol (e.g., propylene glycol, polyethylene glycol), non-ionic surfactant [e.g., polysorbate80, HCO-50 (polyoxyethylene (50 mol) adduct of hydrogenated castor oil)] and the like. As the oily solution, sesame oil, soybean oil and the like are used and, as a solubilizing agent, benzyl benzoate, benzyl alcohol and the like may be used in combination. A prepared injection is preferably filled in a suitable ampoule.

The dose of the agent of the present invention to a subject is not particularly limited as long as it is a therapeutically and/or prophylactically effective amount, and may be appropriately optimized according to the kind and form of the active ingredient, the age and body weight of the subject, the administration schedule, the administration method, and the like.

The timing of administering the agent for the treatment of the present invention to a subject is not particularly limited as long as an ischemic disease can be treated. Examples of the timing of administering the agent for the treatment of the present invention includes, but are not limited to, immediately after the onset of the ischemic disease, and within 1 hr, within 2 hr, within 3 hr, within 4 hr, within 5 hr, within 6 hr, within 7 hr, within 8 hr, within 9 hr, within 10 hr, within 11 hr, within 12 hr, within 24 hr, within 36 hr, within 48 hr, within 72 hr, within 96 hr, and within 120 hr, after the onset of the ischemic disease.

The agent for the treatment of the present invention can also be used in combination with other therapeutic agents for treating ischemic diseases. For example, the agent for the treatment of the present invention may be used in combination with thrombolytic agents (t-PA, urokinase, etc.), anticoagulators (heparin, argatroban, etc.), antiplatelet agents (ozagrel sodium, aspirin, etc.), cerebroprotective agents (edaravone, etc.), anti-cerebral edema agents (glycerol, mannitol, etc.), that are used for the treatment of infarction.

By applying the agent for the treatment of the present invention to a subject affected with an ischemic disease, AIM or AIM fragment is delivered to an infarcted region in the subject, or expressed in an infarcted region in the subject. AIM or AIM fragment in the infarction binds to dead cell debris and/or DAMPs present in the infarction, promotes removal of the dead cell debris, as well as suppresses the spread of sterile inflammation. As a result, the ischemic disease in the subject can be treated.

The "treatment" of a disease in the present specification may include not only cure of a disease, but also remission of the disease and improvement of the degree of the disease.

### 2. Agent for the suppression of sterile inflammation

The present invention also provides an agent for suppressing sterile inflammation mediated by damage-associated molecular patterns (DAMPs), that contains an apoptosis inhibitor of macrophage (AIM), an AIM fragment having a biological activity of AIM, or a nucleic acid encoding the AIM or AIM fragment (hereinafter sometimes to be referred to as "the agent for the suppression of the present invention").

AIM and the like to be the active ingredients in the agent for the suppression of the present invention are similar to those in "1. Agent for the treatment of an ischemic disease". The amounts of AIM and the like to be contained in the agent for the suppression of the present invention are not particularly limited as long as they can suppress sterile inflammation in a subject.

When the active ingredient of the agent for the suppression of the present invention is an AIM protein or a functional fragment thereof, it binds to DAMPs to neutralize the biological activity of DAMPs and/or cause efficient phagocytosis of DAMPs by microglia/macrophage, thus suppressing the spread of sterile inflammation. Examples of the DAMPs to which AIM or a functional fragment thereof binds include, but are not limited to, PRDX1, PRDX2, PRDX3, PRDX4, PRDX5, PRDX6, HMGB1, S100A8, S100A8/9, S100A9, and HSP70. When the active ingredient of the agent for the suppression of the present invention is a nucleic acid encoding AIM or a functional fragment thereof, the AIM or a functional fragment thereof encoded by the nucleic acid is translated in the cell to produce an AIM protein or a functional fragment thereof, thus achieving the desired effect.

The subject to which the agent for the suppression of the present invention is administered is not particularly limited as long as sterile inflammation mediated by DAMPs can occur in the subject. Examples of the subject to which the agent for the suppression of the present invention is administered include, but are not limited to, warm-blooded animals (e.g., human, mouse, rat, rabbit, sheep, swine, bovine, horse, cat, dog, monkey, chimpanzee, bird, and the like). In one embodiment, the subject is a human.

The timing of administering the agent for the suppression of the present invention to a subject is not particularly limited as long as the desired effect can be obtained. Examples of the timing of administering the agent for the suppression of the present invention includes, but are not limited to, immediately after the onset of the inflammation, and within 1 hr, within 2 hr, within 3 hr, within 4 hr, within 5 hr, within 6 hr, within 7 hr, within 8 hr, within 9 hr, within 10 hr, within 11 hr, within 12 hr, within 24 hr, within 36 hr, within 48 hr, within 72 hr, within 96 hr, and within 120 hr, after the onset of the inflammation.

The agent for the treatment of the present invention can also be used in combination with other anti-inflammatory agents for sterile inflammation.

### 3. Method for treating an ischemic disease

The present invention also provides a method for treating an ischemic disease, including administering an apoptosis inhibitor of macrophage (AIM), an AIM fragment having a biological activity of AIM, or a nucleic acid encoding the AIM or AIM fragment to a subject affected with the ischemic disease (hereinafter sometimes to be referred to as "the treatment method of the present invention").

The treatment method of the present invention is achieved by administering the agent for the treatment of the present invention to a subject affected with an ischemic disease. AIM and the like, subject of administration, timing of administration, and the like in the treatment method of the present invention are the same as those in "1. Agent for the treatment of an ischemic disease".

### 4. Method for suppressing a sterile inflammation

A method for suppressing a sterile inflammation mediated by damage-associated molecular patterns (DAMPs), including administering an apoptosis inhibitor of macrophage (AIM), an AIM fragment having a biological activity of AIM, or a nucleic acid encoding the AIM or AIM fragment to a subject affected with the sterile inflammation mediated by DAMPs (hereinafter sometimes to be referred to as "the suppression method of the present invention") is provided.

The suppression method of the present invention is achieved by administering the agent for the suppression of the present invention to a subject affected with sterile inflammation mediated by DAMPs. AIM and the like, subject of administration, timing of administration, and the like in the suppression method of the present invention are the same as those in "2. Agent for the suppression of a sterile inflammation".

The present invention is explained more specifically in the following Examples; however, the present invention is not limited in any way by these Examples.

### [Example]

### [Experiment procedures]

### Mice

To generate AIM^{-/-} mice on a pure C57BL/6 background, two types of the pX335 vectors (Addgene, MA, USA) carrying 5'-caccgaacaatggagccatggccc-3' (SEQ ID NO:3) or 5'-caccggtgagtgtccctgcttctg-3' (SEQ ID NO:4) were microinjected into the pronuclei in fertilized eggs of C57BL/6 mice, and thereafter, the 2-cell embryos were transferred into the uterus of pseudo-pregnant female mice. Genomic DNA isolated from the tail of progenies was tested for any deletion within the cd5l (AIM) gene locus by PCR and sequencing. An AIM^{-/-} mouse line carrying an appropriate deletion at the first ATG region was expanded and used form experiments. Mice were maintained under an SPF condition at the University of Tokyo. Macrophage-specific MafB-deficient mice obtained by crossbreeding of Lysm-Cre transgenic mice and MafB^{flox/flox} mice were maintained under a semi-SPF condition at Tsukuba University, Japan. All animal experiments were carried out in strict accordance with the recommendations in the Guide for the Care and Use of Laboratory Animals of the National Institutes of Health. The protocol was approved by the Committee on the Ethics of Animal Experiments of the University of Tokyo (Permit Number: P10-143). All surgeries were performed under sodium pentobarbital anesthesia, and all efforts were made to minimize suffering.

### Induction of ischemic infarction (MCAO)

Male mice at 8-14 weeks of age and with 20 to 25 g body weight were used for MCAO. According to the procedure of Shichita et al. (2017, Non Patent Literature 9), a silicone-rubber coated nylon monofilament (Doccol) was inserted into the middle cerebral artery laterally for 45 min under an anaesthesia (isoflurane; Pfizer). The cerebral blood flow was monitored using the laser doppler flowmetry (OMEGA) during the MCAO treatment, and mice exhibiting more than 70% reduction in the blood flow at the temporal lobe were used for the following experiments.

### Antibodies and reagents

Antibodies and reagents used for histological experiments are as follows:
Primary antibodies are: AIM (rab2 rabbit polyclonal for IHC of mouse and human brain specimens); #35 (for mouse AIM ELISA), #6 (for human AIM ELISA) established in our laboratory, partly purchasable from Transgenic Inc.), HMGB1 (clone GT383, Gene-Tex, LA, USA), PRDX1 (rabbit polyclonal antibody, abcam, Cambridge, UK), S100A9 (AF2065, R&D systems, NE, USA), Doublecortin (Dcx; chicken polyclonal, ab153668, abcam, Cambridge, UK), Iba1 (goat polyclonal antibody, abcam, Cambridge, UK), biotinylated 6xHis tag (D291-6, MBL, Japan), NF-κB p65 (D14E12, Cell signaling technology), phospho-NF-κB p65 (93H1, Cell signaling technology), and CD11b microbeads (clone: M1/70.15.11.5, Miltenyi Biotec. Bergisch gladbach, Germany). Secondary antibodies and related reagents are: Alexa fluor 488 or 594 conjugated anti-rabbit or rat IgG (Molecular Probes), antihuman IgG-Fc-HRP (A80-104P, Bethyl laboratories, Inc.), Streptavidin-Alexa fluor 488 (Molecular Probes), Streptavidin-HRP (554066, BD Pharmingen), DAPI or Hoechst33342 (Molecular Probes), G-Block (Genostaff, Tokyo, Japan) and HISTOFINE simple stain mouse MAX-PO (R) (for nucleus; NICHIREI, Japan). Specimens were analysed using a confocal microscope: FV10i-DOC and a research slide scanner: SLIDEVIEW VS200 (Olympus, Tokyo).

### DAMPs and related proteins

Mouse PRDXs with a HA tag that were used in the binding assays were generated in house. They were produced in HEK293T cells and thereafter purified from cell lysates using an anti-HA antibody column. Other DAMPs and related proteins were purchased as follows.

Mouse PRDX1-His (RPF757Mu01, USCN), human PRDX1-His (NBC118543, Novus Biologies), mouse S100A8 (9877-S8-050, R&D Systems), mouse S100A9 (2065-S9-050, R&D Systems), mouse S100A8/A9 (8916-S8-050, R&D Systems), human S100A8 (9876-S8-050, R&D Systems), human S100A9 (9254-S9-050, R&D Systems), human S100A8/A9 (8226-S8-050, R&D Systems), mouse HSP70-A1 (low endotoxin; ADI-ESP-502-D, Enzo), human HSP70 (Endotoxin Free; SPR-117A, StressMarq), human HMGB1 (1690-HMB-050, R&D Systems), mouse TLR2-Fc (1530-TR, R&D Systems), human RAGE-Fc (1145-RG, R&D Systems).

### CD11b⁺ cell isolation from brain

Brain dissociation: Mice were deeply narcotized with isoflurane and injected transcardially with 1x phosphate buffered saline (PBS), and their blood was removed. Brain tissues were excised and the infarcted or non-infarcted side of the brains were cut into small pieces, then the pieces were treated with the enzyme solution containing 2.5 U/mL Collagenase D, 8.5 U/mL Dispase, 25 mg/mL DNase I and Complete Mini (Roche; Basel, Switzerland) in 1xHBSS at 37°C for 1 h. The digested tissue was passed through a18 G-needle several times and filtrated through a 70-um strainer (Miltenyi Biotec., Bergisch gladbach, Germany), thereafter centrifuged at 400 g for 15 min at 4°C. The cell-pellets were resuspended in 35% Percoll in PBS and centrifuged at 800 g for 45 min at 4°C. The pellets were resuspended in 0.5% fetal bovine serum (FBS) in PBS. Finally, CD11b+ cells within the total cells were isolated using the CD11b Microbeads (Miltenyi Biotec. Bergisch gladbach, Germany), according to the manufacturer's protocol.

### EILSA for AIM

All ELISA assays were performed in duplicated manner. Mouse AIM in the brain tissue lysate was measured by ELISA using two different rat anti-mouse AIM monoclonal antibodies (rat IgG, clone #35; generated in our laboratory). The inter-assay coefficients of variation assessed by using a C57BL/6 mouse serum was 4.8% for mouse AIM, and the intra-assay coefficients of variation was always less than 4.1%. The lower limit of quantification assessed by using recombinant AIM protein as a standard was 0.0625 ng/ml for mouse AIM.

### AIM mutant proteins

The 2CS mutant was created by substituting the cysteine at 194 to serine (TGC to TCC in nucleotides) in mouse AIM. The ΔSRCR3 mutant was generated by deleting amino acids of the SRCR3 domain (from the aspartic acid at 242 to the valine at 352).

### Purification of rAIM

CHO-S cells were transfected with pcDNA3.1-mAIM plasmid and cultured in CD Forti CHO medium (Invitrogen, CA) for 3 days. rAIM was purified from culture supernatant using rat anti-mouse AIM monoclonal antibody (prepared in our Lab.) conjugated Protein G sepharose (GE Healthcare Life Sciences, PA). Bound protein was eluted with 0.1 M Glycin-HCl, pH 3.0 and neutralized with 1 M Tris-HCl, pH 8.5. Protein was concentrated as necessary using Amicon Ultra filter concentrators (Millipore, MA), and stored at -80°C in PBS. Endotoxin levels were measured by the chromogenic LAL endotoxin detection system (Genscript, NJ) following the manufacturer's protocols. Protein concentration was determined by the BCA (bicinchoninic acid) assay according to the manufacturer's protocol (Pierce, Rockford, IL). Recombinant AIM/IgM-Fc and IgM-Fc proteins were generated as described previously (Hiramoto et al., 2018, The IgM pentamer is an asymmetric pentagon with an open groove that binds the AIM protein. Sci. Adv. 4, eaau1199.).

### Evaluation of binding efficiency of AIM to DAMPs

96-well ELISA plates were coated with DAMPs and control proteins resolved in Bicarbonate buffer (0.1 M NaHCO₃/Na₂CO₃, pH 9.6) at 2 µg/mL unless otherwise specified O/N at 4°C. After the plates were washed with tris buffered saline-tween (TBS-T) four times, they were coated with blocking buffer (10 Casein/TBS) for 2 h at RT. After a wash with TBS-T, AIM resolved in a dilution buffer (0.2% Casein/TBS/2 mM CaCl₂) at various concentrations was added to the well and plates were incubated for 1 h at RT. After 5-times wash with TBS-T/2 mM CaCl₂, biotinylated anti-mouse or human AIM antibody (clone #35 for mouse, clone 7 for human) was added to the wells and incubated for 1 h at RT. After 4-times wash with TBS-T/2 mM CaCl₂, Streptavidin-HRP diluted in 0.2% Casein/TBS/2 mM CaCl₂ was added to the wells and incubated for 1 h at RT. After 4-times wash with TBS-T/2 mM CaCl₂, TMB was added and incubated at RT for 10-25 min. The reaction was stopped by adding 1 N H₂SO₄ and thereby the absorbance at OD 450 nm was analyzed using a multiple plate reader.

### In vitro evaluation of DAMPs captures by macrophage

Peritoneal macrophages isolated from AIM^{-/-}mice were plated on a Lab-Tek II chamber slide (NalgeNunk) and cultured for 2 h to let them attach to the slide. The cells were incubated with 10 µg/mL of DAMPs (mouse PRDX1-His, human PRDX1-His, human HMGB1, or mouse S100A9) with or without wild-type mouse or human rAIM (100 µg/mL) at 37°C for 10 min. For mouse PRDX1, the mutants of 2CS or ΔSRCR3 were also used. Each ligand and AIM were immunostained with antibodies to His (for mouse and human PRDX1), HMGB1 or S100A9, followed by fluorescent-conjugated secondary antibodies. Nuclei were stained with 4',6-diamidino-2-phenylindole (DAPI). The cells were analyzed under a florescence confocal microscope.

### NFκB activation assay

RAW264.1 cells were incubated with mouse S100A9 or RPDX (5 µg/mL) resolved in DMEM+0.1% FBS in the presence or absence of mouse rAIM (10 µg/mL) in 96-well cell culture plates for 30 min at 37°C. After a wash with PBS, cells were lysed using by loading buffer containing SDS, boiled and loaded on an SDS-PAGE gel. Thereafter, the phosphorylation of NFκB was analyzed by immunoblotting using anti-phospho- NFκB p65 antibody (3033S, Cell Signaling Technology, Inc.). Total NFκB was also analyzed using an anti- NFKB antibody (8242S, Cell Signaling Technology, Inc.).

### histology

AIM detection and other IHC at infarcted brain: Brain tissue was excised and fixed in 4% PFA in PBS for 24 h, thereafter embedded in paraffin. 6 um sections were immunostained with the rabbit anti-AIM polyclonal antibody (Rab2; available for human and mouse AIM), followed by incubation with HITOFINE simple stain mouse MAX-PO (R) (NICHIREI, Japan) for 30 min. After stained with diaminobenzidine tetrahydrochloride (DAB), sections were counter-stained with hematoxylin. To block autofluorescence, slides were incubated with 0.5% Sudan Black B (199664-25G, SIGMA-ALDRICH) diluted with 70% ethanol for 25 min before immunostained.

DAMPs staining: Sections were immunostained with a rabbit anti-PRDX1 polyclonal antibody (ab41906), mouse anti-HMGB1 monoclonal antibody (GTX628834), or goat anti-S100A9 polyclonal antibody (AF2065), followed by incubation with HISTOFINE simple stain mouse MAX-PO (R, M, or G) for 30 min.

MAP2 and Iba1 detection: Sections were immunostained with a mouse anti-MAP2 monoclonal antibody (M9942) or goat anti-Iba1 polyclonal antibody (ab5076), followed by incubation with HISTOFINE simple stain mouse MAX-PO (M or G) for 30 min. Before the reaction with primary antibodies, sections were treated in each condition for antigen retrieval. S100A9, HMGB1, and MAP2: Boiled Tris/EDTA buffer (pH 9.0) containing 0.05% Tween-20 for 20 min. PRDX1: 20 µg ProK in PBS at 37°C for 20 min. Iba1: Boiled citrate buffer (10 mM, pH 6.0) containing 0.05% Tween-20 for 20 min. After stained with DAB, sections were counter-stained with hematoxylin.

### Oil red-O staining:

Brain tissues were fixed in 4% paraformaldehyde (PFA) in PBS for 24 h and 30% sucrose solution for 24~48 h and were frozen with OCT compound. 10 µm sections were stained with Oil red-O solution (MUTO OURE CHEMICALS CO., LTD.; Tokyo, Japan). The histologic data for human brain AIM were purchased from Genostaff Co. Ltd. (Tokyo, Japan), who purchased a commercially available paraffin block of a brain tissue from human stroke patient from FUNAKOSHI Co. Ltd. (Japan) and stained the serial sections for human AIM and MAP2.

### Quantification of DAMPs in brain

DAMPs-DAB staining images were obtained and digitally captured using slide scanner VS200 (OLYMPUS, Germany) with a 20x objective lens. Digital image analysis was performed with commercial software HALO (IndicaLabs, Corrales, NM, USA). DAB and hematoxylin signals were detected using object colocalization-based algorithms, and the amount of extracellular DAMPs were estimated by subtracting the hematoxylin-colocalizing.DAB signals from the total DAM signals.

### Evaluation of neurologic deficits (neurologic score)

Behavioural assessments were performed every 24 h after MCAO. The neurologic deficits were scored as previously described (Jiang et al., 2005. Chlortetracycline and demeclocycline inhibit calpains and protect mouse neurons against glutamate toxicity and cerebral ischemia. J. Biol. Chem. 280, 33811-33818.). The criteria is summarized as follows: 0 point: normal; 1 point: mild turning behaviour with/without inconsistent curling when picked up by tail, <50% attempts to curl to the contralateral side; 2 points: mild consistent curling,>50% attempts to curl to contralateral side; 3 points: strong and immediate consistent curling, mouse holds curled position for more than 1-2 seconds, the nose of the mouse almost reaches tail; 4 points: severe curling progressing into barrelling, loss of walking or righting reflex; 5 points: comatose or moribund.

### Statistical analysis

Data were analyzed using BellCurve for Excel (Social Survey Research Information Co., Ltd.) and presented as mean values ± s. d. unless otherwise specified. All data were analyzed by two-sided test. Paired results were assessed using Welch's t-test. Comparisons between multiple groups were analyzed using one-way or multi-way ANOVA followed by the Bonferroni's post hoc test. For the Kaplan-Meier curves, P values were determined with the Generalized Wilcoxon test. Unless otherwise specified, the significance code is as added in each Figure legend.

### Quantitative PCR assay

The quantitative evaluation of mRNA was performed by the ΔΔC_{T} method using a QuantStudio 3 Real-Time PCR system (Thermo Fisher Scientific). Sequences of the oligonucleotides used are listed below.

| Name | Sequence(5'→3') |
|---|---|
| f-GAPDH | AGAACATCATCCCTGCATTC (SEQ ID NO: 5) |
| r-GAPDH | CACATTGGGGGTAGGAACAC (SEQ ID NO: 6) |
| f-mAIM | GAGGACACATGGATGGAATGT (SEQ ID NO: 7) |
| r-mAIMA | CCCTTGTGTAGCACCTCCA (SEQ ID NO: 8) |
| f-IL1B | CAGGCAGGCAGTATCACTCA (SEQ ID NO: 9) |
| r-IL1B | AGGCCACAGGTATTTTGTCG (SEQ ID NO: 10) |
| f-IL6 | ATGGATGCTACCAAACTGGAT (SEQ ID NO: 11) |
| r-IL6 | TGAAGGACTCTGGCTTTGTCT (SEQ ID NO: 12) |
| f-TNFA | CTTCTGTCTACTGAACTTCGGG (SEQ ID NO: 13) |
| r-TNFA | TGATCTGAGTGTGAGGGTCTG (SEQ ID NO: 14) |
| f-IL23 | CAACTTCACACCTCCCTAC (SEQ ID NO: 15) |
| r-IL23 | CCACTGCTGACTAGAACT (SEQ ID NO: 16) |
| f-MCP1 | CATCCACGTGTTGGCTCA (SEQ ID NO: 17) |
| r-MCP1 | GATCATCTTGCTGGTGAATGAGT (SEQ ID NO: 18) |
| f-CD11b | CAATAGCCAGCCTCAGTGCC (SEQ ID NO: 19) |
| r-CD11b | GAGCCCAGGGGAGAAGTGG (SEQ ID NO: 20) |
| f-beta-actin | CTAAGGCCAACCGTGAAAAG (SEQ ID NO: 21) |
| r-beta-actin | ACCAGAGGCATACAGGGACA (SEQ ID NO: 22) |
| f-18SrRNA | CGCCGCTAGAGGTGAAATTCT (SEQ ID NO: 23) |
| r-18SrRNA | CATTCTTGGCAAATGCTTTCG (SEQ ID NO: 24) |

### [Example 1] Increase of brain AIM after onset of ischemic infarction

Expression of AIM(=cd51) mRNA was not detected in the brain of healthy mice but a significant increase in AIM mRNA expression was observed by quantitative PCR (QPCR) on the infarcted side of the brain of mice with lateral infarction caused by transient middle cerebral artery occlusion (MCAO) (Fig. 1A). Furthermore, by analyzing cells that are isolated from the brain on the side where the infarction occurred and are positive and negative for CD11b which is a cell surface marker of macrophage-type cells, it was clarified that AIM mRNA, which increased in the brain on the infarcted side, was expressed by CD11b-positive microglia and macrophages that had infiltrated into the infarct area (Fig. 1A). When lysates prepared by dissolving the brain tissue on day 7 after MCAO were evaluated using enzyme-linked immunosorbent assay (ELISA), no increase in AIM protein was observed in the brain on the non-infarcted side. However, in the infarcted side of the brain, 250 ng at maximum of AIM protein was present per 1 mg of the brain tissue (Fig. 1B). The increase in the expression of AIM in such infarcted brains was evaluated using immunohistochemical staining (IHC). At day 7 after MCAO, AIM was strongly stained in the region of Microtubule-associated protein 2 (MAP2)-negative neurons that were killed by infarction (Fig. 1C). Such accumulation of AIM in the infarcted area was also observed in the brains of human patients with cerebral infarction (Fig. 1D). When observed at high magnification, AIM was strongly stained in Iba1-positive macrophage-like cells (indicated by arrows) in mouse and human brains (Figs. 1C, 1D), and this supports the QPCR results in Fig. 1A showing increased AIM mRNA expression in CD11b-positive macrophage-type cells. Iba1-positive macrophages infiltrating the infarcted area (MAP2 negative) are strongly stained with oil-red-O, indicating that they actively phagocytose dead cell debris (Fig. 1E). In contrast, in AIM-deficient (AIM^{-/-}) mice subjected to MCAO, Iba1-positive macrophages in the infarcted region were mostly oil-red-O negative, indicating that they could not phagocytose dead cell debris (Fig. 1 E). That is, this suggests that AIM contributes to the phagocytic removal of dead cell debris in the infarcted area.

### [Example 2] AIM reduces brain inflammation after infarction caused by DAMPs, and improves prognosis of cerebral infarction

IHC analysis of brain infarcted by MCAO was performed to find that the amount of extracellular PRDX1 (not colocalized with cell bodies and nuclei) was significantly higher in the infarcted area of AIM^{-/-} mice as compared with the brains of wild-type mice. Furthermore, intravenous administration of 0.5 mg of rAIM per mouse once a day from immediately after MCAO reduced PRDX1 in both wild-type and AIM^{-/-} mice (Fig. 2A). It is known that Blood-Brain-Barrier (BBB) is temporarily and partially destroyed during the acute phase of ischemic infarction (55. Zou, R. et al., (2015). Electroacupuncture pretreatment attenuates Blood-Brain Barrier disruption following cerebral ischemia/reperfusion. Mol. Med. Rep. 12, 2027-2034.; Choi, K.H. et al., (2016). Overrexpression of caveolin-1 attenuates brain edema by inhibiting tight junction degradation. Oncotarget. 7, 67857-67867.). In fact, when 0.5 mg of rAIM was transvenously administered to AIM-/- mice 3 days after MCAO, the infarcted brain tissue was widely stained with anti-AIM antibody, indicating that the administered rAIM passed through BBB (Fig. 5). The correlation between the decrease in PRDX1 and the amount of AIM in the brain was evident on days 3 and 7 after MCAO (Fig. 2A, graph). Many AIM^{-/-} mice rapidly lost health and died before day 3 after MCAO. The infarcted region immediately before their deaths (day 2 to 3) had a significantly large amount of S100A9 (Fig. 2B). That is, in a certain number of AIM^{-/-} mice after the onset of infarction, large amounts of DAMPs accumulated in their brains and the mice died. Wild-type mice did not die before day 3. The expression level of inflammatory cytokine mRNA in CD11b-positive macrophages infiltrated into the infarcted brain decreased by rAIM administration in both AIM^{-/-} mice and wild-type mice (Fig. 2C). That is, it is suggested that AIM decreases inflammation in the infarcted region. No significant difference was found in the expression level of inflammatory cytokine mRNA in macrophages in the infarcted area between wild-type mice without administration of rAIM and AIM^{-/-} mice (Fig. 2C). This is considered to be because AIM in the brain has not yet increased in wild-type mice before day 7 (Fig. 1A). Correlating with the amount of DAMPs in the brain and the inflammatory status, the mortality rate at day 7 after MCAO was significantly higher in AIM^{-/-} mice than in wild type mice, but daily transvenous administration of rAIM at 0.5 mg per animal markedly decreased the mortality rate (Fig. 2D). None of the wild-type mice administered with a relatively small amount of rAIM (0.1 mg per mouse) died after MCAO (Fig. 2D). Similar to the improvement in survival rate, neural symptom (hemiplegia) after infarction was more severe in AIM^{-/-} mice than in wild-type mice, but the symptom was re-improved by rAIM administration (Fig. 2E). From the above, intravenous administration of rAIM during the acute phase of infarction remarkably improves the prognosis. That is, it is considered that AIM expressed in the brain or AIM that was administered transvenously and reached the infarcted area promotes the removal of dead cell debris and DAMPs in the infarcted area, reduces inflammation in the brain after infarction, and improves prognosis.

### [Example 3] AIM promoted phagocytic removal of DAMPs, and neutralized DAMPs via direct binding.

In order to verify this hypothesis regarding the action of AIM, the present inventors first confirmed using an ELISA system whether AIM directly binds to DAMPs in the same way as it does to dead cell debris. As shown in Fig. 3A and Fig. 4, AIM bound to PRDX1, S100A9, and HMGB1, as well as many other DAMPs, in a dose-dependent manner. Most DAMPs are negatively charged (Uchida et al., 2014. Natural antibodies as a sensor of electronegative damage-associated molecular patterns (DAMPs). Free Radic. Biol. Med. 72, 156-161.; Knoopset al., 2018. Specific Interactions Measured by AFM on Living Cells between Peroxiredoxin-5 and TLR4: Relevance for Mechanisms of Innate Immunity. Cell Chem. Biol. 25, 550-559.e3.). Therefore, similar to the binding of AIM to dead cell debris (Arai et al., 2016; Tomita et al., 2017), it is considered that AIM may bind to DAMPs through charge-based interactions. To confirm this possibility, the present inventors verified the binding of DAMPs to a mutant mouse AIM (ΔSRCR3) that lacks the carboxy terminal of AIM containing a cluster of positively-charged surface amino acids. As expected, ΔSRCR3 did not show substantial binding to any DAMPs (Fig. 3B). In addition, when the charge-based interactions were inhibited in the presence of high doses of NaCl, the binding between AIM and DAMPs decreased significantly (Fig. 3C). Furthermore, the binding of AIM to DAMPs was inhibited in the presence of the reducing agent tris(2-carboxyethyl)phosphine (TCEP) (Fig. 3D), suggesting further involvement of disulfide bond. The isolated cysteine residue present in the SRCR2 domain of AIM is the only amino acid residue that can be used for the formation of a disulfide bond with another molecule (Miyazaki et al., (1999), Increased susceptibility of thymocytes to apoptosis in mice lacking AIM, a novel murine macrophage-derived soluble factor belonging to the scavenger receptor cysteine-rich domain superfamily. J. Exp. Med. 189, 413-422.; Hiramoto et al., (2018) (the aforementioned)). Therefore, it is highly likely that the cysteine residue forms a disulfide bond with DAMPs. In order to verify this possibility, the present inventors produced another mouse AIM mutant (2CS) in which the cysteine was replaced with serine, and evaluated the binding to DAMPs. As shown in Fig. 3E, the 2CS mutant lost the ability to bind to all of the DAMPs confirmed. Therefore, AIM is considered to stably bind to DAMPs through two independent binding modes, namely, charge-based interaction and disulfide bond formation. These two binding sites are also used to bind AIM and IgM pentamer, as previously reported by the present inventors (Hiramoto et al., (2018) (the aforementioned)). This fact suggests that AIM loses the ability to bind to DAMPs when it is bound to IgM pentamer. In fact, the complex of AIM and IgM pentamer showed almost no binding to DAMPs (Fig. 3F). Furthermore, IgM pentamer not bound to AIM inhibited the binding of AIM to DAMPs in a dose-dependent manner (Fig. 3G). That is, the binding to IgM pentamer functionally inactivates AIM in the binding to DAMPs. Furthermore, the present inventors evaluated under a fluorescence microscope whether the trap and uptake of PRDX1, HMGB1, and S100A9 by macrophages in vitro is inhibited in the presence of rAIM. As shown in Fig. 3H, added rAIM strongly co-localized with all types of DAMPs, supporting the binding data obtained by ELISA (Fig. 3A). Furthermore, the amounts of PRDX1, HMGB1, and S100A9 present on the cell surface or inside the cells significantly increased in the presence of rAIM. This result suggests that rAIM promotes the trap and uptake of DAMPs by macrophages (Fig. 3I). Interestingly, AIM binding strongly induced aggregation of PRDX1 (Fig. 3J), and this recalls AIM-induced bacterial aggregation (Sarrias et al., (2005). A role for human Sp alpha as a pattern recognition receptor. J. Biol. Chem. 280, 35391-35398.). It is known that somewhat larger molecules are more easily taken up by phagocytes (Aderem & Underhill, (1999). Mechanisms of Phagocytosis in Macrophages. Annu. Rev. Immunol. 17, 593-623.; Sarrias et al., (2005) (the aforementioned); Canton et al., Scavenger receptors in homeostasis and immunity. Nat. Rev. Immunol. 13, 621-634.). Therefore, induction of PRDX1 aggregation by AIM is further advantageous for PRDX1 phagocytosis. Both the ΔSRCR3 AIM mutant and the 2CS AIM mutant (Figs. 3B, 3E), which do not bind to DAMPs, did not induce PRDX1 aggregation (Fig. 3J). Further evaluation using an ELISA-based assay showed that binding to AIM itself inhibited the binding of PRDX1 and HMGB1 to TLR2 and RAGE (Fig. 3K). This result suggests that the binding of AIM to DAMPs not only enhances the uptake of DAMPs by phagocytes, but also competitively prevents the charge-based interaction of DAMPs with TLR and RAGE. Such actions of AIM result in a decrease in the inflammatory activity of DAMPs as a whole. As an actual experimental example, as shown in Fig. 3L, the ability of PRDX and S100 to activate NFκB in macrophage cells significantly decreases by the addition of rAIM.

As shown in the aforementioned experimental results, AIM suppresses the inflammatory response by phagocytically removing and neutralizing various DAMPs in the infarcted region caused by ischemia, and promotes efficient removal of dead cell debris, whereby AIM can treat cerebral infarction. It is clear that this action mechanism of AIM can be applied not only to the treatment of cerebral infarction but also ischemic diseases including myocardial infarction. Therefore, the present invention can be used as a therapeutic agent for ischemic diseases.

### [Industrial Applicability]

According to the present invention, ischemic diseases including cerebral infarction can be treated. Therefore, the present invention is extremely beneficial in the medical field.

This application is based on a patent application No. 2021-125789 filed in Japan (filing date: July 30, 2021), the contents of which are incorporated in full herein.

## Claims

1. An agent for the treatment of an ischemic disease,
comprising an apoptosis inhibitor of macrophage (AIM), an AIM fragment having a biological activity of AIM, or a nucleic acid encoding the AIM or AIM fragment.

2. The agent according to claim 1, wherein the ischemic disease is selected from the group consisting of cerebral infarction, myocardial infarction, limb ischemia, pulmonary infarction, splenic infarction, intestinal infarction, and Buerger's disease.

3. An agent for the suppression of sterile inflammation mediated by damage-associated molecular patterns (DAMPs), comprising an apoptosis inhibitor of macrophage (AIM), an AIM fragment having a biological activity of AIM, or a nucleic acid encoding the AIM or AIM fragment.

4. The agent according to claim 3, wherein the DAMP is at least one selected from the group consisting of PRDX1, PRDX2, PRDX3, PRDX4, PRDX5, PRDX6, HMGB1, S100A8, S100A8/9, S100A9, and HSP70.

5. A method for treating an ischemic disease, comprising administering an apoptosis inhibitor of macrophage (AIM), an AIM fragment having a biological activity of AIM, or a nucleic acid encoding the AIM or AIM fragment to a subject affected with the ischemic disease.

6. The method according to claim 5, wherein the ischemic disease is selected from the group consisting of cerebral infarction, myocardial infarction, limb ischemia, pulmonary infarction, splenic infarction, intestinal infarction, and Buerger's disease.

7. A method for suppressing sterile inflammation mediated by damage-associated molecular patterns (DAMPs), comprising administering an apoptosis inhibitor of macrophage (AIM), an AIM fragment having a biological activity of AIM, or a nucleic acid encoding the AIM or AIM fragment to a subject affected with the sterile inflammation mediated by DAMPs.

8. The method according to claim 7, wherein the DAMP is at least one selected from the group consisting of PRDX1, PRDX2, PRDX3, PRDX4, PRDX5, PRDX6, HMGB1, S100A8, S100A8/9, S100A9, and HSP70.
